(19) Europäisches Patentamt
European Patent Office
Office européen des brevets

(11) EP 3 068 756 B1

(12) # FASCICULE DE BREVET EUROPEEN

(45) Date de publication et mention de la délivrance du brevet:
**13.06.2018 Bulletin 2018/24**

(21) Numéro de dépôt: **14806041.1**

(22) Date de dépôt: **07.11.2014**

(51) Int Cl.:
***C07C 67/347*** (2006.01) ***C11C 3/00*** (2006.01)
***C07C 69/716*** (2006.01) ***C11C 3/12*** (2006.01)

(86) Numéro de dépôt international:
**PCT/FR2014/052860**

(87) Numéro de publication internationale:
**WO 2015/071580 (21.05.2015 Gazette 2015/20)**

(54) **HYDROFORMYLATION DE TRIGLYCÉRIDES EN MILIEU AUTO-ÉMULSIFIANT**

HYDROFORMYLIERUNG VON TRIGLYCERIDEN IN EINEM SELBSTEMULGIERENDEN MEDIUM

HYDROFORMYLATION OF TRIGLYCERIDES IN A SELF-EMULSIFYING MEDIUM

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **12.11.2013 FR 1361018**

(43) Date de publication de la demande:
**21.09.2016 Bulletin 2016/38**

(73) Titulaires:
- **Pivert**
  **60280 Venette (FR)**
- **Centre National de la Recherche Scientifique (C.N.R.S.)**
  **75794 Paris Cedex 16 (FR)**
- **Université d'Artois**
  **62030 Arras Cedex (FR)**

(72) Inventeurs:
- **HAPIOT, Frédéric**
  **F-59710 Ennevelin (FR)**
- **MONFLIER, Eric**
  **F-59110 La Madeleine (FR)**
- **VANBESIEN, Théodore**
  **F-59000 Lille (FR)**

(74) Mandataire: **McDade, Sophie V.G.A.**
**Cabinet LLR**
**11, boulevard de Sébastopol**
**75001 Paris (FR)**

(56) Documents cités:
**WO-A2-2008/156993 FR-A1- 2 729 385**

- **ROBERT FRANKE ET AL: "Applied Hydroformylation", CHEMICAL REVIEWS, vol. 112, no. 11, 14 novembre 2012 (2012-11-14), pages 5675-5732, XP055091930, ISSN: 0009-2665, DOI: 10.1021/cr3001803 cité dans la demande**
- **HAPIOT FREDERIC ET AL: "Rhodium-catalyzed hydroformylation promoted by modified cyclodextrins: Current scope and future developments", CURRENT ORGANIC SYNTHESIS, BENTHAM SCIENCE PUBLISHERS LTD, US, vol. 5, no. 2, 1 mai 2008 (2008-05-01), pages 162-172, XP008170559, ISSN: 1570-1794, DOI: 10.2174/157017908784221585**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

EP 3 068 756 B1

**Description**

Domaine technique

L'invention porte sur un procédé d'hydroformylation de triglycérides par création de systèmes permettant l'auto-émulsification temporaire du milieu réactionnel.

Art antérieur

La réaction d'hydroformylation des huiles végétales présente un intérêt majeur au regard des nombreuses applications des produits de réaction qu'elle fournit. Les aldéhydes ainsi formés sont utilisés dans le domaine industriel notamment dans les lubrifiants ou les plastifiants. Cependant, la principale utilisation de ces polyaldéhydes reste leur utilisation en chimie des polymères, notamment pour la synthèse de polymères biosourcés. De plus, l'hydroformylation est reconnue pour être un procédé respectueux de l'environnement car elle permet de limiter la quantité de résidus, voire de les supprimer totalement, tous les atomes constitutifs des réactifs étant impliqués dans les produits finaux.

La réaction d'hydroformylation des huiles végétales et des triglycérides insaturés est effectuée en présence d'un catalyseur et de monoxyde de carbone et d'hydrogène gazeux suivant la formule réactionnelle suivante, où x, y et z sont des nombres entiers, généralement allant de 0 et 22 et où, au moins un des nombres $y_1$, $y_2$ et $y_3$ est différent de 0 et où pour chaque série $x_n$, $y_n$ et $z_n$ (n = 1, 2 ou 3), au moins un de ces nombres est supérieur ou égal à un :

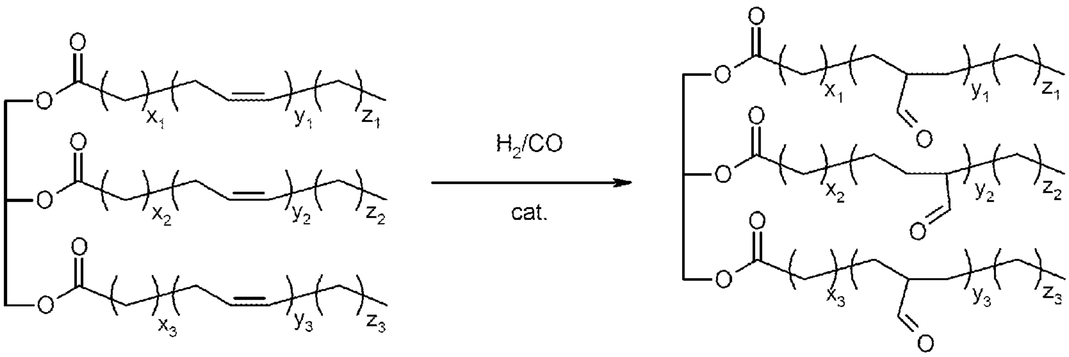


Cette réaction ce déroule le plus souvent en milieu homogène et requiert des catalyseurs généralement à base de cobalt ou de rhodium qui sont le plus souvent associés à des ligands comme des phosphines, par exemple la triphénylphosphine (TPP).

L'article « Applied Hydroformylation », Franke et al. Chemical Reviews (2012), volume 122, No.11, p5675-5732 décrits de tels procédés qui offrent de bons résultats en termes de conversion, de rendement et de sélectivité. Cependant, l'utilisation d'un milieu homogène possède un désavantage majeur. En effet le recyclage du catalyseur nécessite des étapes supplémentaires souvent coûteuses.

Afin de pallier au problème majeur que constitue le recyclage du catalyseur, la réaction d'hydroformylation des huiles végétales et des triglycérides a été transposée en milieu biphasique. Le but est d'immobiliser le catalyseur dans une phase non miscible avec les réactifs, en utilisant par exemple des liquides ioniques ou une phase aqueuse. Dans le cas des phases aqueuses, des additifs tels que des tensioactifs ou de la silice sont nécessaires pour favoriser les contacts entre le substrat à transformer et le catalyseur. L'utilisation de ces additifs complique cependant le recyclage du catalyseur.

Dans ce contexte, il a été reporté que les cyclodextrines (CD) pouvaient être des additifs efficaces en catalyse d'hydroformylation biphasique. FR2729385 décrit l'utilisation d'un catalyseur rhodié tel que $Rh(acac)(CO)_2$ en présence de la triphénylphosphine trisulfonée (TPPTS) et de CD éventuellement substituées, pour des réactions d'hydroformylation d'oléfines linéaires ou ramifiées de taille moyenne en milieu biphasique sans former d'émulsion. Seul le 1-décène y est exemplifié. En fin de réaction, les produits décantent permettant la récupération des deux phases.

L'Article « Aqueous hydroformylation reaction mediated by randomly methylated β-cyclodextrin : How substitution degree influences catalytic activity and selectivity », F.-X Legrand et al, Journal of Molecular Catalysis A : Chemical (2009), volume 303, No.1-2, p72-77, étudie l'influence du degré de méthylation sur l'activité catalytique de la réaction d'hydroformylation du 1-décène. Plus précisément y sont étudiés différents types de cyclodextrines méthylées présentes dans un produit commercial, la RAME-β-CD, dans lequel le degré de substitution moyen par unité glucosidique (DS) varie de 1,4 à 1,9. L'augmentation de l'activité catalytique augmente proportionnellement au DS et un DS de 1,9 apparait être le plus performant.

L'article « Biphasic aqueous organometallic catalysis promoted by cyclodextrins : can surface tension measurements explain the efficiency of chemically modified cyclodextrins?», L. Leclerc et al., Journal of Colloid and Interface Science (2007) volume 307, No.2, p481-487, décrit l'effet positif de la méthylation et de l'hydroxypropylation de CD sur la tension interfaciale de systèmes biphasiques lors de la catalyse homogène de la réaction d'hydroformylation d'oléfines à chaîne courte (comme par exemple des $C_{10}$). Les cyclodextrines HP-β-CD (DS moyen de 0,6 et 0,8) ainsi que RAME-β-CD (DS moyen 1,8) font parties des CDs testées et se montrent actives pour des réactions d'hydroformylation.

Les complexes d'inclusion entre les molécules organiques et les CD peuvent avoir des propriétés tensioactives intéressantes permettant la mise en émulsion du système réactionnel. Dans l'article « Versatile Eco-friendly Pickering Emulsions Based on Substrate/Native Cyclodextrine complexes : A wining approach for Solvent-Free oxidations », Leclercq et al, ChemSusChem (2013), volume 6, No.8, p1533-1540, sont décrites des réactions d'oxydation catalytique de substrats organiques en présence de CD native. L'émulsion stable résultante est stabilisée par les complexes substrat/CD possédant des propriétés tensioactives. La récupération de la phase organique et le recyclage de la phase aqueuse nécessitent des étapes de centrifugation et chauffage.

L'article « Structure of inclusion Complexes of Cyclodextrins with Triglyceride of vegetable Oil/Water interface », K. Shimada et al. Journal of Food science (1992) volume 57, No.3, p655, décrit des complexes d'inclusion formés entre des triglycérides et des α-CD ou β-CD non substituées, ceci dans des systèmes biphasiques. Ces complexes permettent l'obtention de systèmes émulsionnés stables.

De plus WO 02/100524 A1 montre également que les complexes corps gras/CD peuvent présenter des propriétés tensioactives et que ces complexes peuvent être isolés. Les triglycérides sont cités dans la liste des corps gras envisagés mais ne sont pas exemplifiés.

## Description de l'invention

Il est maintenant proposé un procédé d'hydroformylation de triglycérides qui combine un taux de conversion et une sélectivité élevés tout en permettant une grande simplicité d'extraction des produits de la réaction, ceci dans des conditions opératoires douces et en milieu aqueux.

Selon un mode de réalisation, l'invention porte sur un procédé d'hydroformylation de triglycérides par catalyse homogène en présence d'au moins une cyclodextrine substituée (CD) par un groupe méthyle ou hydroxypropyle, ledit procédé comprenant une étape a) de mise en présence sous agitation d'au moins un catalyseur, d'eau, d'au moins un triglycéride insaturé ayant plus de 45 atomes de carbone et de ladite cyclodextrine substituée, en présence d'hydrogène gazeux et de monoxyde de carbone, ladite étape étant réalisée dans des conditions réactionnelles permettant l'établissement d'une émulsion lors de l'agitation et une décantation des produits après l'arrêt de l'agitation; lesdites conditions réactionnelles comprenant notamment une agitation mécanique allant de 400 à 1400 tours/minute et l'étape a) étant effectuée sous pression d'un gaz comprenant de l'hydrogène moléculaire et du monoxyde de carbone a une pression supérieure, ou égale, à 20 bars.

## Définitions

Triglycéride : triester dérivé du glycérol et de trois acides gras, saturés ou insaturés, de longueur de chaine grasse variable mais comportant généralement au moins 14 atomes de carbone. Dans le cadre de l'invention les triglycérides insaturés utilisé dans le procédé sont des composés comportant plus de 45 atomes de carbones. Les triglycérides utilisables dans le procédé de l'invention peuvent adopter la formule décrite précédemment. De préférence les séries de nombres $x_n$, $y_n$ et $z_n$ sont identiques. Il est également préféré que les chaines grasses des triglycérides insaturés comprennent chacune au moins 14 atomes de carbones, de préférence 16, 18 ou plus de 20 atomes de carbones. Les triglycérides insaturés sont généralement très hydrophobes.

Catalyse homogène : procédé catalytique où le catalyseur est soluble dans au moins une phase liquide.

Cyclodextrine (CD): oligosaccharide cyclique contenant respectivement 6, 7 ou 8 unités glucopyranoses (respectivement nommé α, β ou γ) liée en α-(1,4). Les cyclodextrines peuvent être natives (sans modification) ou substituées par différents groupements chimiques d'intérêt (méthyle, hydroxypropyle...) sur les différentes positions hydroxyles de la molécule. Dans ce dernier cas la substitution peut être totale ou partielle. Le degré de substitution est déterminé par RMN $^1$H et spectrométrie de masse (MALDI, electrospray).

Conditions réactionnelles : conditions opératoires dans lesquelles se déroule la réaction. Ces conditions sont, par exemple, les concentrations et/ou les proportions relatives des réactifs mis en présence ainsi que les caractéristiques physiques du milieu telles que la pression, la température et l'agitation.

Emulsion : dispersion macroscopiquement homogène, microscopiquement hétérogène, de deux substances non miscibles dont la stabilité varie dans le temps.

Conditions normales d'agitation : Le système réactionnel est agité mécaniquement dans une gamme de 400 à 1400 tours/minute. La vitesse d'agitation est généralement fixée à sa valeur maximale pour une dispersion optimale

de la phase organique dans la phase aqueuse.

Décantation rapide : un des aspects particulièrement avantageux de l'invention est une décantation rapide, c'est-à-dire une séparation des phases aqueuses et huileuse (organique) en moins d'une heure, de préférence en moins de 30 min et avantageusement en moins de 10 min (par exemple moins de 5 min).

Détermination des conditions d'obtention d'une émulsion active en catalyse.

Pour pouvoir déterminer les conditions d'obtention d'une émulsion permettant de réaliser l'invention il convient d'étudier le pouvoir auto-émulsifiant du couple CD/triglycérides et d'avoir ainsi un aperçu macroscopique de l'état du système eau/triglycéride/CD. Une méthode expérimentale connue est d'établir un diagramme de phases en observant une série de mélanges dont seules les proportions respectives de CD, de triglycérides et d'eau varient et en fixant les autres conditions, telles que la pression, la température, l'agitation etc. Après arrêt de l'agitation, l'évolution du milieu est alors observée au cours du temps. Ces expériences permettent de déterminer par observation le domaine de proportions des réactifs à utiliser pour les conditions données qui permettent d'obtenir l'émulsion requise, domaine dans lequel il convient de se placer pour réaliser l'invention. L'étendue de ces domaines est fonction de la durée après laquelle le système est observé après agitation. Une durée de deux minutes a été choisie afin de pouvoir évaluer l'état du système eau/huile/CD dans des conditions jugées proches des conditions initiales de la catalyse, c'est à dire avec des températures allant de 25 à 100 °C, de préférence 80°C, et une agitation de 1400 tr/min. Ainsi pour un triglycéride tel que la trioléine, les domaines suivants ont été observés :

Domaine A : deux phases distinctes, une phase aqueuse et une phase huileuse
Domaine B : émulsion (huile dans l'eau) + une phase aqueuse
Domaine C : phase comportant un solide en suspension
Domaine D : émulsion (huile dans l'eau)

Le domaine B est celui dans lequel il convient de se placer pour effectuer la réaction car il permet d'obtenir une séparation de phases en fin de réaction. Les conditions du domaine A ne permettent pas d'obtenir une réaction d'hydroformylation effective. De même il est possible de réaliser des diagrammes de phases pour d'autres conditions de la réaction d'hydroformylation que les concentrations respectives des réactifs. Ces autres conditions peuvent être, par exemple, la température ou la pression du mélange. L'élaboration de diagrammes de phases est bien connue et formalisée et fait partie des connaissances de base de l'homme du métier. Par exemple les articles de référence édités par Techniques De l'Ingénieur j2150, intitulé « Emulsion - Elaboration et étude des émulsions » et publié le 12 décembre 2013 d'une part, et j2158, intitulé « Formulation des émulsions par la méthode du HDL » le 10 septembre 2006, d'autre part, établissent bien que cette technique est parfaitement connue et maîtrisée.

Selon un mode de réalisation préféré de l'invention la cyclodextrine substituée utilisée est une cyclodextrine-$\alpha$, -$\beta$ ou -$\gamma$. De préférence ladite cyclodextrine est une cyclodextrine -$\beta$ ou -$\gamma$.

Il est également préféré que la cyclodextrine utilisée dans le procédé selon l'invention soit choisie dans le groupe constituée par RAME- $\beta$-CD, HP-$\alpha$-CD, HP-$\beta$-CD, HP-$\gamma$-CD, RAME-$\gamma$-CD, CRYSMEB et leurs mélanges.

Le degré de substitution moyen de la cyclodextrine substituée peut-être choisi dans une gamme allant de 0,5 à 3, de préférence allant de 0,5 à 2,0. Lorsque la cyclodextrine est une cyclodextrine méthylée, un degré de substitution moyen d'environs 1,8 $\pm$ 10% ou de 0,8 $\pm$ 10% est particulièrement avantageux. Lorsque la cyclodextrine substituée est une cyclodextrine hydroxypropylée, un degré de substitution moyen variant de 0,5 à 0,9, de préférence de 0,5 à 2,0, comme par exemple 0,6 ou 0, 8, est particulièrement avantageux.

Selon un mode de réalisation particulier du procédé selon l'invention l'étape a) est effectuée à une température choisie dans la gamme allant de 40 et 140°C, de préférence de 40°C à 120°C, plus particulièrement de 50 à 70°C. Une température aux environs de 60°C ou de 80°C, $\pm$ 5°C est préférée.

Il est également préféré que le rapport molaire cyclodextrine/triglycéride soit choisi dans une gamme allant de 0,5 à 3,0, de préférence allant de 0,2 à 4,0 aux environs de 2,9.

Selon un aspect préféré de l'invention, la pression du gaz qui comprend l'hydrogène moléculaire et le monoxyde de carbone est choisie dans une gamme allant de 20 à 200 bars. De préférence la pression dudit gaz comprenant de l'hydrogène moléculaire et du monoxyde de carbone est supérieure, ou égale, à 80 bars

De préférence, le catalyseur de la réaction comprend, ou est constitué d'un métal de transition tel que le rhodium, le ruthénium, le palladium, l'iridium, le cobalt et le platine ou d'un composé bimétallique tel que platine/étain et platine/fer. De manière particulièrement avantageuse ce catalyseur est sous forme de complexe associé à une phosphine, éventuellement sulfonée et/ou hydrosoluble, telle que la TPPTS ou une phényle(s)/ biphényle(s) phosphine sulfonée.

La phosphine peut être une phosphine hydrosoluble répondant à la formule générale suivante :

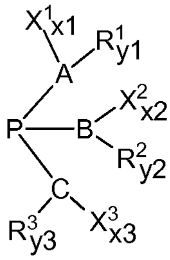

dans laquelle :

- A, B, C, qui peuvent être identiques ou différents, représentent chacun un groupement aryle choisi parmi les groupements phényles, les groupements naphtyles et les groupements biphényles ou parmi les groupements alkyles ayant de 1 à 10 atomes de carbone ;
- les substituants $R^1$, $R^2$, $R^3$, qui peuvent être identiques ou différents, représentent chacun un groupement choisi parmi l'hydrogène, les radicaux alkyles ayant de 1 à 4 atomes de carbone, les radicaux alkoxy ayant de 1 à 4 atomes de carbone, les atomes d'halogène, les groupes -CN,-$NO_2$, -OH et -N(Z1)(Z2), Z1 et Z2, identiques ou différents, représentant chacun un radical alkyle ayant de 1 à 5 atomes de carbone ;
- les substituants $X^1$, $X^2$ et $X^3$, qui peuvent être identiques ou différents, représentent chacun un groupement acide carboxylique, un groupement acide sulfonique ou leurs sels. Lorsque $X^1$, $X^2$ ou $X^3$ représentent un sel d'acide carboxylique ou sulfonique, le cation peut être un cation de métal alcalin ou alcalino-terreux ou un cation ammonium de formule $N(T^1T^2T^3T^4)^+$ dans laquelle $T^1$, $T^2$, $T^3$, $T^4$, qui peuvent être identiques ou différents, représentent chacun un groupe alkyle comportant de 1 à 4 atomes de carbone.
- x1, x2, x3, sont des nombres identiques ou différents, variant entre 0 et 3 inclus, l'un au moins parmi eux étant égal ou supérieur à 1 ;
- y1, y2, y3 sont des nombres identiques ou différents pouvant varier entre 2 et 9 inclus.

De préférence, A, B et C représentent chacun un groupement aryle, plus particulièrement un groupement phényle ou biphényle. Les substituants $X^1$ à $X^3$ sont avantageusement -$SO_3$Na ou -$CO_2$Na. Les substituants $R^1$ à $R^3$ sont avantageusement l'hydrogène ou un groupement alkyle. Les nombres x1, x2 et x3 sont avantageusement égaux à 1 ou 0 (l'un d'entre eux au moins étant égal à 1). Les nombres y1, y2 et y3 peuvent varier entre à 4 et 9.

On peut citer à titre d'exemples, la triphénylphosphine trisulfonée (TPPTS) (A = B = C = phényle ; $R^1 = R^2 = R^3$ = H ; $X^1 = X^2 = X^3 = SO_3Na$ ; x1 = x2 = x3 = 1 ; y1 = y2 = y3 = 4) et la biphényldiphénylphosphine trisulfonée (A = B = phényle ; C = biphényle ; $R^1 = R^2 = R^3$ = H ; $X^1 = X^2 = X^3 = SO_3Na$ ; x1 = x2 = x3 = 1 ; y1 = y2 = 4 ; y3 = 8).

Les phosphines sulfonées particulièrement avantageuses pour le procédé de la présente invention sont la TPPTS et les phényl(s)-biphényl(s) phosphines sulfonées.

Selon un aspect préféré de l'invention ledit triglycéride insaturé est un mélange de triglycérides, lequel mélange ne comprend avantageusement pas plus de 25%, de préférence pas plus de 15% et plus particulièrement pas plus de 10% en mole de chaines grasses polyinsaturés dans les triglycérides, c'est-à-dire de chaînes carbonées comprenant au moins deux double liaisons C=C.

Le triglycéride insaturé est de préférence un triglycéride comprenant un nombre moyen de double liaison C=C inférieur ou égal à 3,5 et de préférence inférieur ou égal à 3. Ce nombre peut par exemple aller de 1 à 3, de préférence de 2 à 2,90 et en particulier de 2,7 à 2,8.

Le procédé selon l'invention peut être effectué à partir d'une huile biosourcée comme des huiles végétales, raffinées ou non, par exemple des huiles d'oléagineux. Les huiles végétales peuvent être par exemple des huiles d'olive, de sésame, de soja, de tournesol, de colza. De préférence ces huiles peuvent être des huiles d'olive ou de tournesol.

Selon un aspect particulièrement avantageux de l'invention, l'étape a) est réalisée en l'absence de solvant organique, et en particulier sans composés organiques ne participant pas à la réaction et/ou étant présent à plus de 10% en poids dans le milieu réactionnel. De préférence, comme ladite décantation permet la séparation d'une phase organique contenant les produits de la réaction, l'étape de récupération des produits de la réaction est effectuée par séparation du milieu réactionnel de ladite phase.

La quantité d'eau du milieu réactionnel est de préférence supérieure ou égale à 50% en masse du milieu réactionnel. Pour certaines CD, peu méthylées (par exemple taux de 0,8) il peut être avantageux de se placer dans un domaine de concentration supérieur ou égal à 65% massique en eau, par exemple 72% massique. Bien évidemment il convient de se placer dans un domaine de concentration permettant une décantation des produits de la réaction tout en évitant une trop grande dilution des produits (par exemple une quantité d'eau supérieure à 75% en masse).

Le temps de réaction peut varier de quelques minutes à plusieurs jours suivant les conditions réactionnelles.

Il est généralement de plusieurs heures mais est généralement inférieur à 24 heures. Il peut être choisi entre 2 et 20 heures, avantageusement entre 4 et 19 heures, par exemple 6 ou 18 heures.

Selon un autre mode de réalisation, l'invention porte sur l'utilisation d'au moins une des cyclodextrines modifiées susmentionnées dans un procédé d'hydroformylation des triglycérides tel que décrit précédemment.

## Brève description des dessins

L'invention sera mieux comprise à la lecture des figures annexées, qui sont fournies à titre d'exemples et ne présentent aucun caractère limitatif, dans lesquelles :

Figure 1 : diagramme de phase à 80°C d'un système eau/trioléine/RAME-β-CD deux minutes après arrêt de l'agitation.
Figure 2 : diagrammes de phase du système eau/trioléine/RAME-β-CD à 25°C, 80°C et 100°C, deux minutes après arrêt de l'agitation.

## Description détaillée de l'invention

**Exemple 1 : Elaboration du diagramme de phase pour le système eau/trioléine/cyclodextrine substituée (CD) à t=2min.**

Ce diagramme est élaboré en faisant varier les proportions massiques entre les composants. Les résultats sont présentés à la Figure 1. Les proportions sont calculées de la façon suivante : $\%(i) = \frac{m_i}{m_{total}} = \left(\frac{m_i}{m_{CD} + m_{eau} + m_{trioléine}}\right)$, $m_i$ étant la masse du composé dont on veut déterminer la proportion.

Les émulsions étant dépendantes de la température, le diagramme a été élaboré à une température de 80°C (température utilisée pour les tests catalytique). Le mode opératoire utilisé est le suivant :
Dans un tube à essai muni d'un agitateur magnétique, on introduit des quantités prédéterminées de RAME-β-CD et de trioléine (cf. Tableau 1a, tubes 1 à 4). On ajoute ensuite une certaine quantité d'eau à chaque tube. Le milieu est laissé sous agitation (barreau magnétique à 1400 tour/min) dans un bain d'huile à 80 °C pendant 20 min. L'agitation est alors arrêtée. Deux minutes après l'arrêt de l'agitation, le tube à essai est retiré du bain pour observation. Une fois les phases identifiées, une nouvelle quantité d'eau est ajoutée, changeant ainsi les proportions des différents constituants.

Les opérations décrites ci-dessus sont répétées jusqu'à obtention des résultats présentés dans le tableau 1b qui sont portées sur le diagramme de phase et permettent de déterminer les domaines A, B, C et D suivants :

Domaine A : deux phases distinctes, une phase aqueuse et une phase huileuse
Domaine B : émulsion (huile dans l'eau) + une phase aqueuse
Domaine C : phase comportant un solide en suspension
Domaine D : émulsion (huile dans l'eau)

Tableau 1a :

| Masse eau totale (g) | Masse CD (g) | Masse huile (g) |
|---|---|---|
| Tube 1 | 0,1 | 0,36 |
| Tube 2 | 0,3 | 0,46 |
| Tube 3 | 0,5 | 0,27 |
| Tube 4 | 0,7 | 0,14 |

Tableau 1b :

| Exemple 1 | Masse eau totale (g) | 0,1 | 0,2 | 03 | 0,4 | 0,6 | 0,8 | 1 | 1,4 | 1 ,8 | 2,2 | 3,6 |
|---|---|---|---|---|---|---|---|---|---|---|---|---|
| | Domaines | A<br>C | A<br>D | A<br>B | A<br>B | A<br>B | B<br>B | B<br>B | B<br>B | B<br>B | B<br>B | B<br>B |

(suite)

| | | | | | | | | | | | | |
|---|---|---|---|---|---|---|---|---|---|---|---|---|
| Exemple 1 | Masse eau totale (g) | 0,1 | 0,2 | 03 | 0,4 | 0,6 | 0,8 | 1 | 1,4 | 1 ,8 | 2,2 | 3,6 |
| | | C<br>C | C<br>C | B<br>D | B<br>D | B<br>D | B<br>B | B<br>B | B<br>B | B<br>B | B<br>B | B<br>B |

**Exemple 2 : Réaction d'hydroformylation d'un triglycéride, la trioléine, dans un système auto-émulsifiant selon l'invention.**

Le schéma réactionnel est le suivant :

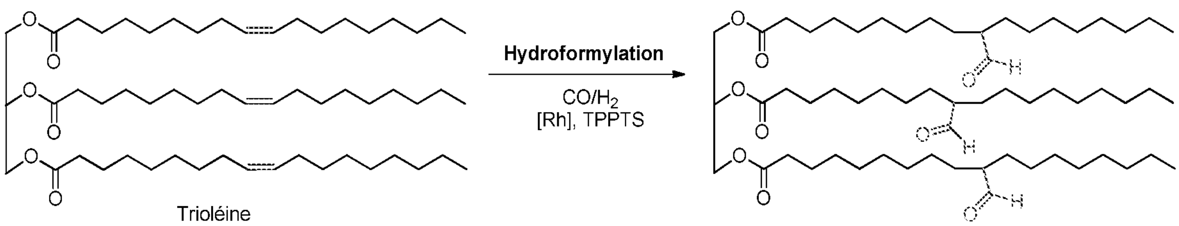


Trioléine

Dans un autoclave de 25 mL contenant 1,9 mmol (1,8 mL) de trioléine (soit 5,8 mmol de doubles liaisons C=C internes), on introduit 6,2 mL d'une solution aqueuse contenant 3,05 mmol (4 g) de β-cyclodextrines partiellement méthylées (RAME-β-CD) de masse molaire moyenne 1310 $g.mol^{-1}$ et de degré de substitution moyen de 1,8 par unité glucosidique, 0,029 mmol (7,5 mg) d'(acétylacétonato)dicarbonyl rhodium(I) ainsi que 0,15 mmol (85 mg) de sel tri-sodique de tri(méta-sulfonatophenyl)phosphine.

Ce système correspond aux proportions données en Figure 1 sur le diagramme de phase. L'autoclave est chauffé à 80 °C, pressurisé sous 50 bars d'un mélange CO/H2 en proportion équimolaire, l'agitation est réglée à 1400 tours/minute pendant 18 heures.

Après arrêt de l'agitation on observe une phase émulsionnée contenant l'huile (produits initiaux et produits de réaction) dans l'eau et une phase aqueuse. Cinq minutes après l'arrêt de l'agitation la phase émulsionnée a totalement décanté permettant l'extraction de la phase organique et le recyclage de la phase aqueuse contenant le catalyseur et les CDs.

Une conversion de 26% et une sélectivité de 78% en faveur de l'aldéhyde sont déterminées par RMN des produits de réaction après décantation de ces derniers. Cette conversion correspond à une conversion des insaturations contenues dans la trioléine et non pas d'une conversion de la trioléine elle-même.

Sélectivité en aldéhyde : nombre de mole d'aldéhyde formé/nombre de mole d'oléfines initiales qui ont été converties.

**Exemples 3 à 7 : influence de la quantité de CD substituée sur la réaction d'hydroformylation de l'exemple 2.**

Ces exemples ont été réalisés dans les mêmes conditions expérimentales en temps, pression et température ainsi qu'avec les mêmes agents catalytiques que l'exemple 2. Ils montrent l'influence de la quantité de CD sur la réaction d'hydroformylation.

Les positions relatives des exemples 4 à 7 sur le diagramme de phase établi précédemment sont données en Figure 1. L'expérience 3 est un exemple sans cyclodextrine et est donné à titre comparatif. On peut montrer que le domaine B est une zone permettant d'avoir une conversion et une sélectivité significative. En effet, l'exemple 7, situé dans le domaine D, montre une baisse de la sélectivité de l'hydroformylation. Les conditions expérimentales particulières ainsi que les résultats sont rassemblés dans le tableau 2 ci-dessous.

Tableau 2 :

| Exemples | Masse de RAME-β-CD (g) | Rapport molaire RAME-β-CD/ triglycérides | Conversion des doubles liaisons (%) | Sélectivité en aldéhyde (%) |
|---|---|---|---|---|
| 3 | 0 | /// | 0 | /// |
| 4 | 0,5 | 0,20 | 5 | 62 |
| 5 | 2 | 0,82 | 19 | 84 |
| 6 | 7 | 2,88 | 39 | 78 |

(suite)

| Exemples | Masse de RAME-β-CD (g) | Rapport molaire RAME-β-CD/ triglycérides | Conversion des doubles liaisons (%) | Sélectivité en aldéhyde (%) |
|---|---|---|---|---|
| 7 | 9 | 3,71 | 35 | 44 |

**Exemples 8 à 12: influence de la température sur la réaction d'hydroformylation de l'exemple 2**

Les exemples suivants ont été réalisés dans les mêmes conditions expérimentales et avec les mêmes agents catalytiques que l'exemple 2. Ces exemples montrent l'influence de la température de réaction sur l'activité du système catalytique inhérente à l'invention. La température influençant l'émulsion, plusieurs diagrammes de phases élaborés à des températures de 25 °C, 80 °C et 100°C (à t=2min) sont représentés en Figure 2. La réaction a lieu dans le domaine B pour ces trois températures. Les conditions de température ainsi que les résultats sont rassemblés dans le tableau 3 :

Tableau 3 :

| Exemple | Température (°C) | Conversion des doubles liaisons (%) | Sélectivité en aldéhyde (%) |
|---|---|---|---|
| 8 | 25 | 21 | 75 |
| 9 | 40 | 47 | 90 |
| 10 | 60 | 49 | 86 |
| 11 | 100 | 21 | 48 |
| 12 | 120 | 11 | 28 |

Ces résultats montrent un domaine d'application en température compris entre 40 °C et 80 °C avec un optimum de température à 60 °C pour une conversion et une sélectivité optimales. Considérant que la température d'hydroformylation usuelle est généralement aux environs de 80 °C, ce résultat est inattendu et particulièrement avantageux puisqu'il permet de réaliser la réaction dans des conditions plus douces en minimisant les dépenses d'énergie.

**Exemples 13 à 14** : **Influence de la pression sur la réaction d'hydroformylation de l'exemple 2.**

Les exemples suivants ont été réalisés dans les conditions expérimentales identiques à l'exemple 2. Les exemples suivants reflètent l'influence de la pression en gaz de synthèse $CO/H_2$ sur la conversion et la sélectivité. Les résultats de ces exemples sont rassemblés dans le tableau 4 :

Tableau 4 :

| Exemple | Pression (bar) | Conversion des doubles liaisons (%) | Sélectivité en aldéhyde (%) |
|---|---|---|---|
| 13 | 20 | 12 | 25 |
| 14 | 80 | 58 | 87 |

Ces résultats nous montrent l'effet de la pression sur l'activité du système inhérent à l'invention.

**Exemples 15 à 21 : Comparaison entre les CDs substituées utilisées dans le procédé selon l'invention et d'autres CDs substituées**

Les exemples suivants ont été réalisés avec diverses CDs natives ou substituées dans les mêmes conditions expérimentales que l'exemple 2 et après vérifications que ces conditions réactionnelles sont celles située dans le domaine B et permettent donc d'obtenir une émulsion. Ils permettent de montrer l'influence de la cyclodextrine et des modifications chimiques apportées à celles-ci sur la conversion (Conv) et la sélectivité (Sélec) en aldéhyde. Les résultats sont rassemblés dans le tableau 5.

Tableau 5 :

| Exem -ple | Modification | DS[c] | poids $_{CD}$(%)[d] | T (°C) | P (bar) | Conv (%) | Sélec (%) | Décan -tation |
|---|---|---|---|---|---|---|---|---|
| 15 | α-CD | // | 5 | 80 | 50 | >2 | /// | Non |
| 16 | β-CD | // | 7 | 80 | 50 | 3 | /// | Non |

(suite)

| Exem -ple | Modification | DS[c] | poids $_{CD}$(%)[d] | T (°C) | P (bar) | Conv (%) | Sélec (%) | Décan -tation |
|---|---|---|---|---|---|---|---|---|
| 17 | RAME-β-CD[a] | 1,8 | 34 | 80 | 80 | 58 | 87 | Oui |
| 18 | RAME-γ-CD | 1,8 | 34 | 80 | 80 | 32 | 76 | Oui |
| 19 | HP-α-CD[b] | 0,6 | 34 | 80 | 80 | 29 | 81 | Oui |
| 20 | HP-β-CD[b] | 0,8 | 34 | 80 | 80 | 98 | 90 | Oui |
| 21 | HP-β-CD[b] | 0,6 | 34 | 80 | 80 | 95 | 89 | Oui |
| 22 | HP-γ-CD[b] | 0,6 | 34 | 80 | 80 | 86 | 90 | Oui |

[a]correspond à une cyclodextrine partiellement méthylée
[b] correspond à une cyclodextrine partiellement substituée avec un groupement 2-(hydroxypropyl)
[c] DS : degré de substitution par unité glucosidique
[d] proportion massique de la cyclodextrine en question dans le milieu réactionnel

**Exemple 23 à 27 : procédé d'hydroformylation sur un mélange de triglycérides d'origine naturel : huile d'olive commerciale**

Les exemples suivants ont été réalisés dans les mêmes conditions que l'exemple 2. Comme dans les exemples précédents, on a préalablement vérifié que le système se trouve dans un domaine de type B par élaboration des diagrammes de phase. On observe pour l'huile commerciale, comme pour la trioléine, une décantation après réaction. Ces huiles végétales ne sont pas pures mais les résultats suivants permettent de dire que la présente invention est adaptée pour l'hydroformylation de mélanges complexes de triglycérides. Les résultats sont rassemblés dans le tableau 6.

Tableau 6 :

| Exemple | Cyclodextrine | Pressi on (bar) | Température (°C) | Temps (heures) | Conversion (%) | Sélectivit é (%) |
|---|---|---|---|---|---|---|
| 23 | RAME-β-CD (1,8)[a] | 50 | 80 | 18 | 44 | 56 |
| 24 | RAME-β-CD (1,8)[a] | 80 | 80 | 18 | 66 | 70 |
| 25 | RAME-β-CD (1,8)[a] | 100 | 50 | 6 | 35 | 78 |
| 26 | RAME-β-CD (1,8)[a] | 150 | 50 | 6 | 58 | 81 |
| 27 | HP-β-CD (0,6)[a] | 80 | 80 | 18 | 78 | 85 |

[a] Le nombre entre parenthèse représente le degré de substitution par unité glucosidique de la cyclodextrine en question.

L'huile commerciale utilisée pour ces exemples a été analysée par transestérification des triglycérides dans le méthanol et par chromatographie gazeuse pour déterminer les différents dérivés d'acide gras composant l'huile végétale. Les résultats sont regroupés dans le tableau 7 :

Tableau 7 :

| Acide gras[a] | Proportion molaire dans l'huile d'olive (%) |
|---|---|
| Acide palmitoléique ($C_{16:1}$) | 0,7 |
| Acide palmitique ($C_{16:0}$) | 11,1 |
| Acide stéarique ($C_{18:0}$) | 3,2 |
| Acide oléique ($C_{18:1}$) | 76,7 |
| Acide linoléique ($C_{18:2}$) | 4,1 |
| Acide linolénique ($C_{18:3}$) | 1,7 |
| Acide arachidique ($C_{20:0}$) | 2,5 |

[a] Les abréviations entre parenthèse permettent de schématiser le type d'acide gras. Le premier nombre représente le nombre total d'atome de carbone de l'acide gras, le deuxième nombre indiquant le nombre d'insaturations sur la chaine carbonée.

**Exemple 28 à 31 : Influence de la nature de la phosphine**

Les exemples suivants ont été réalisés selon les mêmes conditions expérimentales que l'exemple 2, hormis la nature de la phosphine utilisée en association avec le rhodium ainsi que la pression en gaz de synthèse CO/H2 qui est de 80 bars. On utilise dans ces exemples une monophosphine sulfonée avec un rapport molaire phosphine/métal de 5/1. Les phosphines utilisées ainsi que les effets de ces dernières sur la réaction d'hydroformylation sont donnés en tableau 8 :

Tableau 8 :

| Exemple | Phosphine | Structure de la phosphine | Conversion (%) | Sélectivité (%) |
|---|---|---|---|---|
| 28 | Tris(o-toluyl) phosphine disulfonée | 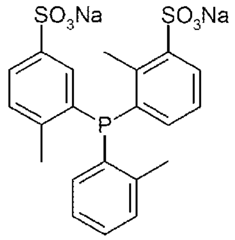 | 91 | 88 |
| 29 | Biphényldiphenyl phosphine trisulfonée | 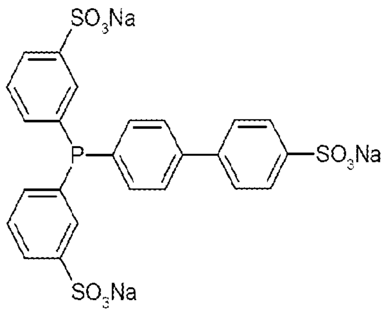 | 58 | 85 |
| 30 | Bis(biphényl)phenyl phosphine trisulfonée | 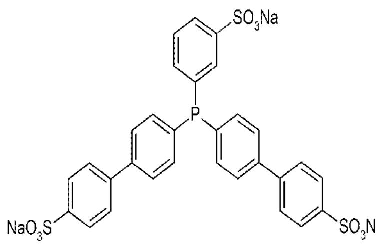 | 72 | 86 |
| 31 | Tris(biphényl) Phosphine trisulfonée | 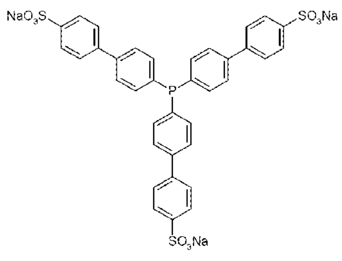 | 51 | 82 |

Les phosphines particulièrement avantageuses dans le procédé de la présente invention sont la TPPTS et les phényl(s)-biphényl(s) phosphines sulfonées, c'est-à-dire des phosphines sulfonées comprenant des groupements phényles et/ou des groupements biphényles. Les exemples répertoriés dans le tableau 8 permettent de montrer la validité de la présente invention pour une gamme de phosphines aromatiques sulfonées.

**Exemple 32 : Influence de la température pour la HP-β-CD**

La présence d'un optimum de température pour la HP-β-CD est établi en utilisant le mode opératoire de l'exemple 2 dans les conditions réactionnelles particulières suivantes : %massique des différents constituants : HP-β-CD 34 %, eau : 54%, huile 17%, 80 bars $CO/H_2$, 18 heures, 1 mmol de trioléine (3 mmol de doubles liaisons), rapport molaire : double liaison/Rh = 200 ; rapport molaire Phosphine/Rh = 5. Le catalyseur et la phosphine sont ceux de l'exemple 2. Une séparation de phase a bien lieu. Les résultats présentés dans le tableau 9 ont été obtenus par les méthodes d'analyse précédemment décrites.

**Tableau 9**

| Température (°C) | Conversion des doubles liaisons (%) | Sélectivité en aldéhyde (%) |
|---|---|---|
| **30** | 28 | 68 |
| **50** | 53 | 79 |
| **60** | 69 | 85 |
| **80** | 91 | 90 |
| **100** | 55 | 65 |
| **120** | 13 | 23 |

**Exemple 33 : Influence de la concentration massique de HP-β-CD**

Le rôle de la concentration massique de HP-β-CD dans la réaction est établi en utilisant le mode opératoire de l'exemple 2 dans les conditions réactionnelles particulières suivantes : eau : 3,4 mL, pression 80 bars CO/H2, temps d'agitation 18 heures, 1 mmol de trioléine (3 mmol de doubles liaisons), rapport molaire double liaison/Rh= 200, rapport molaire Phosphine/Rh=5. Le catalyseur et la phosphine sont ceux de l'exemple 2. Une séparation de phase a bien lieu sauf lorsque la concentration en CD est en dehors du domaine de séparation de phase désirée. Les résultats présentés dans le tableau 10 ont été obtenus par les méthodes d'analyse précédemment décrites.

**Tableau 10**

| % massique de CD | Conversion des doubles liaisons (%) | Sélectivité en aldéhyde (%) | Décantation |
|---|---|---|---|
| **44** | 87 | 89 | Non |
| **34** | 91 | 90 | Oui |
| **20** | 61 | 84 | Oui |
| **12** | 43 | 70 | Oui |
| **7** | 23 | 59 | Oui |

Ces résultats indiquent que, dans la mesure où l'on se place dans des conditions de séparation de phases permettant la décantation, plus la quantité de cyclodextrine est élevée plus les taux de conversion et de sélectivité sont élevés.

**Exemple 34 : Procédé d'hydroformylation utilisant de la HP-β-CD sur de l'huile d'olive, validation des températures et des concentrations massiques du mélange**

Le rôle de 1) la température, 2) la concentration massique de HP-β-CD et 3) la concentration massique d'eau (à rapport HP-β-CD/huile constant) est établi en utilisant le mode opératoire de l'exemple 2.
1) Pour la température, les conditions réactionnelles particulières sont les suivantes : %massique : HP-β-CD : 34 %, eau : 54%, huile 17%, pression : 80 bars CO/H2, durée de l'agitation 18 heures, 1 mmol d'huile d'olive commerciale, rapport molaire double liaison/Rh= 200, rapport molaire Phosphine/Rh=5. Le catalyseur et la phosphine sont ceux de l'exemple 2. Une séparation de phase a bien lieu. Les résultats présentés dans le tableau 11 ont été obtenus par les méthodes d'analyse précédemment décrites.

**Tableau 11**

| Température (°C) | Conversion des doubles liaisons (%) | Sélectivité en aldéhyde (%) |
|---|---|---|
| **30** | 28 | 68 |
| **50** | 50 | 79 |
| **60** | 55 | 80 |
| **80** | 78 | 85 |

(suite)

| Température (°C) | Conversion des doubles liaisons (%) | Sélectivité en aldéhyde (%) |
|---|---|---|
| **100** | 48 | 65 |
| **120** | 12 | 23 |

Ainsi il est établi un optimum de température aux alentours de 80°C.

2) Pour la concentration massique de HP-β-CD, les conditions réactionnelles particulières sont les suivantes : eau : 3,4 mL, pression 80 bars CO/H2, temps d'agitation 18 heures, 1 mmol d'huile d'olive commerciale, rapport molaire double liaison/Rh= 200, rapport molaire Phosphine/Rh=5. Le catalyseur et la phosphine sont ceux de l'exemple 2. Une séparation de phase a bien lieu sauf lorsque la concentration en CD est en dehors du domaine de séparation de phase désirée. Les résultats présentés dans le tableau 12 ont été obtenus par les méthodes d'analyse précédemment décrites.

**Tableau 12**

| % massique de CD | Conversion des doubles liaisons(%) | Sélectivité en aldéhyde (%) | décantation |
|---|---|---|---|
| **44** | 81 | 76 | Non |
| **34** | 78 | 85 | Oui |
| **20** | 51 | 72 | Oui |
| **12** | 38 | 70 | Oui |
| **7** | 23 | 56 | Oui |

Ces résultats indiquent que, dans la mesure où l'on se place dans des conditions de séparation de phases permettant la décantation, plus la quantité de cyclodextrine est élevée, plus les taux de conversion et de sélectivité sont élevés.

3) Pour la concentration massique d'eau (à rapport massique HP-β-CD/huile d'olive constant et égal à 2) les conditions réactionnelles particulières sont les suivantes : 2.3 g de HP-β-CD, 1.1 mL d'huile d'olive commerciale, pression 80 bars CO/H2, temps d'agitation 18 heures, rapport molaire double liaison/Rh= 200, rapport molaire Phosphine/Rh=5. Le catalyseur et la phosphine sont ceux de l'exemple 2. Une séparation de phase a bien lieu sauf lorsque la quantité d'eau est en dehors du domaine de séparation de phase désirée. Les résultats présentés dans le tableau 13 ont été obtenus par les méthodes d'analyse précédemment décrites.

**Tableau 13**

| % massique d'eau | Conversion des doubles liaisons(%) | Sélectivité en aldéhyde (%) | Décantation |
|---|---|---|---|
| **24** | 3 | 43 | Non |
| **39** | 19 | 54 | Non |
| **52** | 78 | 85 | Oui |
| **66** | 74 | 83 | Oui |
| **79** | 66 | 77 | Oui |
| **88** | 55 | 65 | Oui |

Ces résultats indiquent que, dans la mesure où l'on se place dans des conditions de séparation de phases permettant la décantation, la quantité d'eau optimum est inférieure à 79% en masse.

**Exemple 35: Procédé d'hydroformylation utilisant de la CRYSMEB sur de la trioléine.**

La CRYSMEB est une cyclodextrine β méthylée en position 2 avec un faible taux de substitution ($DS_{CRYSMEB}$=0,8 vs $DS_{RAMEB}$ =1,8). Cette cyclodextrine a été préalablement testée en condition d'émulsion. Elle permet d'obtenir des émulsions un peu plus stable (décantation légèrement plus lente) mais permettant tout de même une décantation de l'huile après un temps de repos hors agitation de l'ordre de quelques minutes.

La présence d'un optimum de température pour la CRYSMEB est établi en utilisant le mode opératoire de l'exemple 2 dans les conditions réactionnelles particulières suivantes : %massique des différents constituants : CRYSMEB 20%, eau : 71%, huile 9% (1 mmol de trioléine (3 mmol de double liaisons)), pression 80 bars CO/H2, temps d'agitation 6 heures, rapport molaire : double liaison/Rh = 200 ; rapport molaire Phosphine/Rh = 5. Le catalyseur et la phosphine sont ceux de l'exemple 2. Une séparation de phase a bien lieu. Les résultats présentés dans le tableau 14 ont été obtenus par les méthodes d'analyse précédemment décrites.

**Tableau 14**

| Température | Conversion des doubles liaisons (%) | Sélectivité en aldéhyde (%) |
|---|---|---|
| **40** | 69 | 87 |
| **60** | 96 | 88 |
| **80** | 96 | 94 |
| **100** | 74 | 84 |
| **120** | 36 | 70 |

Ainsi il est établi un optimum de température aux alentours de 60 à 80°C.

Le rôle de la concentration massique de CRYSMEB dans la réaction est établi en utilisant le mode opératoire de l'exemple 2 dans les conditions réactionnelles particulières suivantes : eau : 8,0 mL, température 80 °C, pression 80 bars $CO/H_2$, temps d'agitation 6 heures, 1 mmol de trioléine (3 mmol de double liaisons), doubles liaisons/Rh= 200, Phosphine/Rh=5. Le catalyseur et la phosphine sont ceux de l'exemple 2. Une séparation de phase a bien lieu. Le catalyseur et la phosphine sont ceux de l'exemple 2. Une séparation de phase a bien lieu sauf lorsque la concentration en CD est en dehors du domaine de séparation de phase désirée. Les résultats présentés dans le tableau 15 ont été obtenus par les méthodes d'analyse précédemment décrites.

**Tableau 15**

| % massique de CD | Conversion des doubles liaisons (%) | Sélectivité en aldéhyde (%) | Décantation |
|---|---|---|---|
| **25** | 94 | 94 | Non |
| **20** | 96 | 94 | Oui |
| **11** | 79 | 97 | Oui |
| **6** | 61 | 85 | Oui |
| **3** | 41 | 82 | Oui |
| **0** | <1 | /// | Oui |

L'étude en fonction de la proportion massique de CD montre que plus la quantité de CD augmente dans le milieu, plus la conversion et la sélectivité augmentent. L'augmentation de la concentration en CD permettrait la formation de plus de complexes d'inclusion par basculement de l'équilibre en faveur de la formation de ce dernier. Le nombre de complexes tensioactifs augmentant, l'émulsion qui en résulte serait plus stable, plus homogène avec une interface maximale. Cependant, il faut noter que, passer une certaine masse de CD, la conversion n'évolue plus et la sélectivité commence à décroitre. De plus, l'émulsion se stabilise de plus en plus avec la proportion de CD ce qui est, bien sûr, à éviter.

**Exemple 36 Procédé d'hydroformylation d'huile de tournesol.**

Le procédé est réalisé en utilisant de l'huile de tournesol et trois cyclodextrines précédemment décrites. Le protocole expérimental est le même que celui de l'exemple 2, dans les conditions particulières suivantes :

[a] Conditions: CD (2.3 g), huile (1 mL, 1 mmol), $Rh(CO)_2(acac)$ (3.9 mg, 0.015 mmol), TPPTS (42 mg, 0.075 mmol), eau (5 mL), pression CO/H2 = 80 bars, température = 80°C, durée d'agitation 18h.

[b] Conditions: CD (2.3 g), huile (1 mL, 1 mmol), $Rh(CO)_2(acac)$ (3.9 mg, 0.015 mmol), TPPTS (42 mg, 0.075 mmol), eau (8 mL), pression CO/H2 = 80 bars, température = 80°C, durée d'agitation 6h.

Les résultats pour obtenus ont été compilés avec ceux précédemment obtenus pour l'huile d'olive afin de comparaison dans le tableau 16 :

| Entrée | CD | Huile | Nombre de doubles liaisons C=C initiales | Conversion des doubles liaisons C=C (%) | Sélectivité en aldéhyde (%) | % initial de polyinsatur é dans l'huile | % d'isomérisation des chaines polyinsaturées |
|---|---|---|---|---|---|---|---|
| **1[a]** | RAME-β-CD | Olive | 2.78 | 48 | 71 | 5.8 | - |
| **2[a]** | HP-β-CD | Olive | 2.78 | 78 | 85 | 5.8 | - |
| **3[a]** | HP-β-CD | Tournesol | 2.75 | 51 | 80 | 7.7 | 39 |
| **4[b]** | CRYSMEB | Olive | 2.78 | 86 | 86 | 5.8 | - |
| **5[b]** | CRYSMEB | Tournesol | 2.75 | 54 | 80 | 7.7 | 52 |

Nombre de doubles liaisons C=C initiales : nombre moyen de doubles liaisons C=C présents initialement dans les triglycérides composant l'huile comprenant tous les types d'insaturations (monoinsaturé + polyinsaturé). Nombre d'insaturations des huiles végétales déterminé par RMN 1H.

% initial de polyinsaturés dans l'huile : pourcentage molaire d'esters gras présentant au moins deux doubles liaisons sur la chaine carbonée (dérivés linoléique, linolénique) dans l'huile en question. Pourcentage obtenus après transestérification méthanolique des triglycérides et analyse en GC et GC-MS des esters méthyliques obtenus.

Pourcentage d'isomérisation des chaines polyinsaturées : nombre de mole d'isomère conjugué formé/nombre de mole de chaines polyinsaturées initiales. Noter que seuls les dérivés polyinsaturés peuvent être isomérisés.

L'invention n'est pas limitée aux modes de réalisations présentées et d'autres modes de réalisation apparaîtront clairement à l'homme du métier.

**Revendications**

1. Procédé d'hydroformylation de triglycérides par catalyse homogène en présence d'au moins une cyclodextrine substituée par un groupe méthyle ou hydroxypropyle, ledit procédé comprenant une étape a) de mise en présence, sous agitation, d'au moins un catalyseur, d'eau, d'au moins un triglycéride insaturé ayant plus de 45 atomes de carbone et de ladite cyclodextrine substituée, en présence d'hydrogène gazeux et de monoxyde de carbone, ladite étape étant réalisée dans des conditions réactionnelles permettant l'établissement d'une émulsion lors de l'agitation et une décantation des produits de la réaction après arrêt de l'agitation ; lesdites conditions réactionnelles comprenant notamment une agitation mécanique allant de 400 à 1400 tours/minute et l'étape a) étant effectuée sous pression d'un gaz comprenant de l'hydrogène moléculaire et du monoxyde de carbone a une pression supérieure, ou égale, à 20 bars.

2. Le procédé selon la revendication 1, **caractérisé en ce que** ladite cyclodextrine substituée est une cyclodextrine-$\alpha$, -$\beta$ ou -$\gamma$.

3. Le procédé selon la revendication 1 ou 2, **caractérisé en ce que** ladite cyclodextrine méthylée présente un degré de substitution moyen allant de 0,5 à 2, de préférence 0,8.

4. Le procédé selon la revendication 1 ou 2, **caractérisé en ce que** ladite cyclodextrine hydroxypropylée présente un degré de substitution moyen variant de 0,5 à 2,0, de préférence 0,8.

5. Le procédé selon l'une quelconque des revendications 1 à 4, **caractérisé en ce que** l'étape a) est effectuée à une température allant de 40°C à 120°C, de préférence de 60°C à 80°C.

6. Le procédé selon l'une quelconque des revendications 1 à 5, **caractérisé en ce que** le rapport molaire cyclodextrine/triglycéride est choisi dans une gamme allant de 0,2 à 4, et de préférence est de 2,9.

7. Le procédé selon l'une quelconque des revendications 1 à 6, **caractérisé en ce que** la pression dudit gaz comprenant de l'hydrogène moléculaire et du monoxyde de carbone est supérieure, ou égale, à 80 bars.

8. Le procédé selon l'une quelconque des revendications 1 à 7, **caractérisé en ce que** ledit catalyseur est un complexe comprenant un métal de transition associé à une phosphine sulfonée.

9. Le procédé selon la revendication 8, **caractérisé en ce que** ladite phosphine sulfonée est la TPPTS ou une phényle(s)/biphényle(s) phosphine sulfonée.

10. Le procédé selon l'une quelconque des revendications 1 à 9, **caractérisé en ce que** ledit triglycéride est un mélange de triglycérides, lequel mélange ne comprend pas plus de 10% en mole de chaînes grasses polyinsaturées dans les triglycérides.

11. Le procédé selon l'une quelconque des revendications 1 à 10, **caractérisé en ce que** lesdites conditions réactionnelles sont proches, ou sont celles, de ladite catalyse réalisée sans la présence de cyclodextrine.

12. Le procédé selon l'une quelconque des revendications précédentes, **caractérisé en ce que** ladite décantation permet la séparation d'une phase organique contenant les produits de la réaction et que l'étape de récupération des produits de la réaction est effectuée par séparation du milieu réactionnel de ladite phase.

## Patentansprüche

1. Verfahren zur Hydroformylierung von Triglyceriden durch homogene Katalyse in Gegenwart mindestens eines Cyclodextrins, substituiert durch eine Methyl- oder Hydroxypropylgruppe, wobei das Verfahren einen Schritt a) des in Gegenwart Bringens, unter Rühren, von mindestens einem Katalysator, von Wasser, von mindestens einem ungesättigten Triglycerid mit mehr als 45 Kohlenstoffatomen und von dem substituierten Cyclodextrin, in Gegenwart von gasförmigem Wasserstoff und von Kohlenmonoxid, umfasst, wobei der Schritt unter Reaktionsbedingungen realisiert wird, die das Bilden einer Emulsion beim Rühren und das Dekantieren der Produkte der Reaktion nach Anhalten des Rührens gestatten; wobei die Reaktionsbedingungen insbesondere ein mechanisches Rühren mit 400 bis 1.400 Umdrehungen/Minute umfassen und Schritt a) unter Druck eines Gases, das molekularen Wasserstoff und Kohlenmonoxid enthält, bei einem Druck von größer oder gleich 20 bar erfolgt.

2. Verfahren nach Anspruch 1, **dadurch gekennzeichnet, dass**, das substituierte Cyclodextrin ein $\alpha$-, $\beta$- oder $\gamma$-Cyclodextrin ist.

3. Verfahren nach Anspruch 1 oder 2, **dadurch gekennzeichnet, dass** das methylierte Cyclodextrin einen gemittelten Substitutionsgrad von 0,5 bis 2, vorzugsweise von 0,8, aufweist.

4. Verfahren nach Anspruch 1 oder 2, **dadurch gekennzeichnet, dass** das hydroxypropylierte Cyclodextrin einen gemittelten Substitutionsgrad von 0,5 bis 2,0, vorzugsweise von 0,8, aufweist.

5. Verfahren nach einem der Ansprüche 1 bis 4, **dadurch gekennzeichnet, dass** Schritt a) bei einer Temperatur von 40 °C bis 120 °C, vorzugsweise von 60 °C bis 80 °C, erfolgt.

6. Verfahren nach einem der Ansprüche 1 bis 5, **dadurch gekennzeichnet, dass** das Molverhältnis Cyclodextrin/Triglycerid in einem Bereich von 0,2 bis 4 gewählt ist und vorzugsweise 2,9 ist.

7. Verfahren nach einem der Ansprüche 1 bis 6, **dadurch gekennzeichnet, dass** der Druck des Gases, das molekularen Wasserstoff und Kohlenmonoxid enthält, größer oder gleich 80 bar ist.

8. Verfahren nach einem der Ansprüche 1 bis 7, **dadurch gekennzeichnet, dass** der Katalysator ein Komplex ist, der ein Übergangsmetall, assoziiert mit einem sulfonierten Phosphin, umfasst.

9. Verfahren nach Anspruch 8, **dadurch gekennzeichnet, dass** das sulfonierte Phosphin TPPTS oder ein sulfoniertes Phenyl-/Biphenylphosphin ist.

10. Verfahren nach einem der Ansprüche 1 bis 9, **dadurch gekennzeichnet, dass** das Triglycerid ein Gemisch aus Triglyceriden ist, wobei das Gemisch nicht mehr als 10 Mol-% polygesättigter Fettsäuren in den Triglyceriden enthält.

11. Verfahren nach einem der Ansprüche 1 bis 10, **dadurch gekennzeichnet, dass** die Reaktionsbedingungen nahe der oder die der Katalyse sind, die ohne die Gegenwart von Cyclodextrin realisiert wird.

12. Verfahren nach einem der vorhergehenden Ansprüche, **dadurch gekennzeichnet, dass** das Dekantieren die Abtrennung einer organischen Phase gestattet, welche die Produkte der Reaktion enthält, und der Schritt des Auffangens der Produkte der Reaktion durch Abtrennung aus einem Reaktionsmedium der Phase erfolgt.

## Claims

1. Process for the hydroformylation of triglycerides by homogeneous catalysis in the presence of at least one cyclodextrin substituted with a methyl or hydroxypropyl group, said process comprising a step a) of bringing together, with stirring, at least one catalyst, water, at least one unsaturated triglyceride having more than 45 carbon atoms and said substituted cyclodextrin, in the presence of hydrogen gas and of carbon monoxide, said step being carried out under reaction conditions allowing the formation of an emulsion during the stirring and decanting of the reaction products once the stirring has stopped; said reaction conditions comprising in particular mechanical stirring ranging from 400 to 1400 revolutions/minute and step a) being carried out under pressure of a gas comprising molecular hydrogen and carbon monoxide at a pressure greater than or equal to 20 bar.

2. Process according to Claim 1, **characterized in that** said substituted cyclodextrin is an α-, β- or γ-cyclodextrin.

3. Process according to Claim 1 or 2, **characterized in that** said methylated cyclodextrin has an average degree of substitution ranging from 0.5 to 2, preferably 0.8.

4. Process according to Claim 1 or 2, **characterized in that** said hydroxypropylated cyclodextrin has an average degree of substitution ranging from 0.5 to 2.0, preferably 0.8.

5. Process according to any one of Claims 1 to 4, **characterized in that** step a) is carried out at a temperature ranging from 40°C to 120°C, preferably from 60°C to 80°C.

6. Process according to any one of Claims 1 to 5, **characterized in that** the cyclodextrin/triglyceride mole ratio is chosen in a range of from 0.2 to 4, and is preferably 2.9.

7. Process according to any one of Claims 1 to 6, **characterized in that** the pressure of said gas comprising molecular hydrogen and carbon monoxide is greater than or equal to 80 bar.

8. Process according to any one of Claims 1 to 7, **characterized in that** said catalyst is a complex comprising a transition metal combined with a sulfonated phosphine.

9. Process according to Claim 8, **characterized in that** said sulfonated phosphine is TPPTS or a sulfonated phenyl(s)/biphenyl(s) phosphine.

10. Process according to any one of Claims 1 to 9, **characterized in that** said triglyceride is a mixture of triglycerides, which mixture does not comprise more than 10 mol% of polyunsaturated fatty chains in the triglycerides.

11. Process according to any one of Claims 1 to 10, **characterized in that** said reaction conditions are close to, or are those of, said catalysis carried out without the presence of cyclodextrin.

12. Process according to any one of the preceding claims, **characterized in that** said decanting allows the separation of an organic phase containing the reaction products and that the step of recovering the reaction products is carried out by separation of the reaction medium from said phase.

# Figure 1

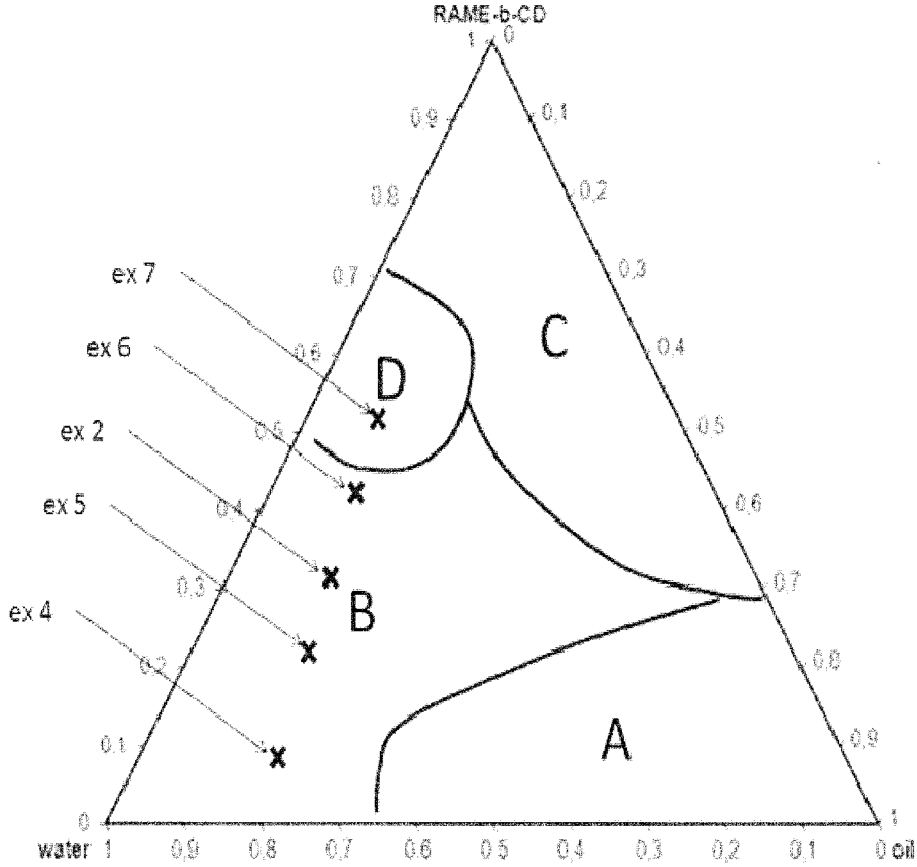
RAME-b-CD
1
0
0,9
0,1
0,8
0,2
0,7
0,3
0,6
0,4
0,5
0,5
0,4
0,6
0,3
0,7
0,2
0,8
0,1
0,9
0
1
water
1
0,9
0,8
0,7
0,6
0,5
0,4
0,3
0,2
0,1
0 oil
ex 7
ex 6
ex 2
ex 5
ex 4
A
B
C
D

Figure 2

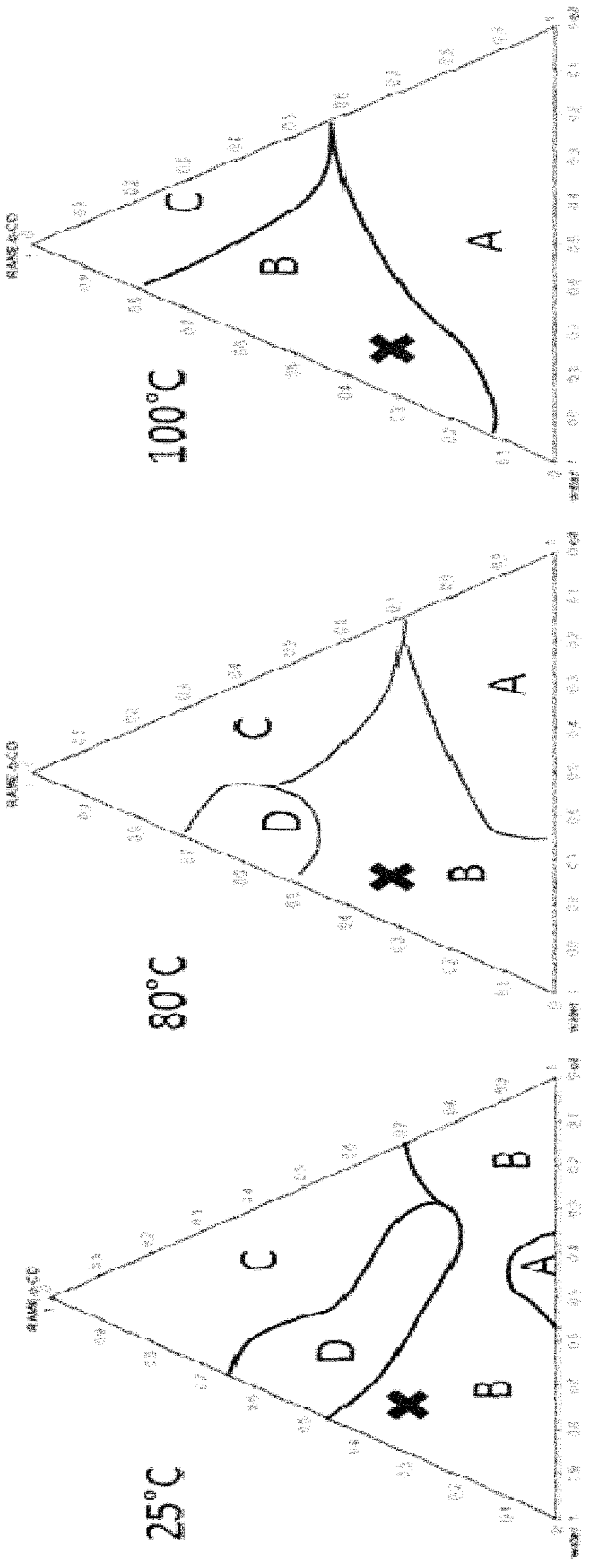

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- FR 2729385
- WO 02100524 A1


### Littérature non-brevet citée dans la description


- **FRANKE et al.** Applied Hydroformylation. *Chemical Reviews,* 2012, vol. 122 (11), 5675-5732
- **F.-X LEGRAND et al.** Aqueous hydroformylation reaction mediated by randomly methylated β-cyclodextrin : How substitution degree influences catalytic activity and selectivity. *Journal of Molecular Catalysis A : Chemical,* 2009, vol. 303, 72-77
- **L. LECLERC et al.** Biphasic aqueous organometallic catalysis promoted by cyclodextrins : can surface tension measurements explain the efficiency of chemically modified cyclodextrins?. *Journal of Colloid and Interface Science,* 2007, vol. 307 (2), 481-487
- **LECLERCQ et al.** Versatile Eco-friendly Pickering Emulsions Based on Substrate/Native Cyclodextrine complexes : A wining approach for Solvent-Free oxidations. *ChemSusChem,* 2013, vol. 6 (8), 1533-1540
- **K. SHIMADA et al.** Structure of inclusion Complexes of Cyclodextrins with Triglyceride of vegetable Oil/Water interface. *Journal of Food science,* 1992, vol. 57 (3), 655
- Emulsion - Elaboration et étude des émulsions. 12 Décembre 2013
- Formulation des émulsions par la méthode du HDL. *j2158,* 10 Septembre 2006